# DEMANDE DE BREVET EUROPEEN

(11) **EP 1 616 551 A1**
(43) Date de publication de la demande: **18.01.2006**
(21) Numéro de dépôt: 05291517.0
(22) Date de dépôt: 13.07.2005
(51) Int. Cl.: A61K 8/02, A61K 8/97, A61K 8/49, A61K 8/34, A61K 8/31, A61K 8/60, A61Q 19/08

(54) **Procédé de traitement cosmétique pour prévenir ou retarder les signes du vieillissement cutané**

(30) Priorité: 13.07.2004 FR 0451518; 13.07.2004 FR 0451521
(71) Demandeur: L'OREAL, 75008 Paris (FR)
(72) Inventeur: Legendre, Jean-Yves, 75015 Paris (FR); Duranton, Albert, 78600 Maison Laffite (FR); Mahe, Yann, 91390 Morsang sur Orge (FR)
(74) Mandataire: Allab, Myriam

(57) **Abrégé**

L'invention concerne un procédé de traitement cosmétique pour prévenir et/ou retarder et/ou limiter les signes du vieillissement de la peau, des muqueuses ou des phanères, caractérisé en ce qu'on applique sur une zone de la peau ou du cuir chevelu un système de délivrance transdermique, la composition dudit système transdermique contenant au moins un micronutriment non indispensable.

## Description

La présente invention concerne des procédés de traitement cosmétique de la peau, des muqueuses et/ou des phanères mettant en oeuvre des dispositifs de délivrance transdermiques et les dispositifs adaptés à ces applications.

En vue d'améliorer l'aspect de la peau ou des phanères, ou de les maintenir en bon état, on utilise classiquement des compositions cosmétiques, qui sont appliquées de manière topique sur la zone où une activité cosmétique est recherchée.
Ces compositions donnent de bons résultats; toutefois, certains actifs, requièrent parfois des formulations spécifiques pour atteindre les cellules cibles, ou ne manifestent leur activité qu'après des applications prolongées et répétées.
On a par ailleurs proposé des compléments nutritionnels pour prévenir ou réduire certaines altérations esthétiques de la peau ou des phanères, en renforçant leur protection endogène. Ces formes orales constituent un bon complément aux formes topiques puisqu'elles permettent aux composés actifs d'atteindre l'ensemble de l'organisme et notamment lorsqu'il s'agit de zones corporelles importantes. Cependant, certains individus considèrent comme une contrainte de devoir ingérer quotidiennement voire même plusieurs fois par jour, souvent pendant une période prolongée, des formes galéniques telles que des gélules, granules, comprimés ou autres, proches de celles utilisées dans le domaine pharmaceutique. Cela les conduit parfois à interrompre la prise de ces compléments avant d'avoir obtenu une efficacité optimale. De plus, certaines molécules subissent, pendant le cycle entéro-hépatique, des transformations susceptibles de réduire leur activité, et ne peuvent donc être administrées efficacement par cette voie.

Il existe donc un besoin de procédé de traitement cosmétique permettant une bonne distribution des agents dans l'ensemble des tissus dont on souhaite améliorer l'aspect, sans dégradation substantielle de la forme active et qui nécessite un nombre d'application limité et/ou l'application sur une surface corporelle restreinte et/ou inférieure à celle où les effets sont attendus.

Ces buts et d'autres sont atteints dans le cadre de la présente invention par un procédé de traitement cosmétique pour améliorer l'aspect de la peau, des muqueuses ou des phanères, et plus spécifiquement pour prévenir et/ou retarder et/ou limiter les signes du vieillissement, caractérisé en ce qu'on applique sur une zone de la peau, des muqueuses, ou du cuir chevelu un système de délivrance transdermique, la composition dudit système transdermique contenant au moins un micronutriment, de préférence au moins un micronutriment non indispensable.

En effet, la demanderesse a maintenant trouvé qu'il est possible de maintenir en bon état et/ou de renforcer la résistance de la peau ou des phanères aux différentes agressions, intrinsèques ou extrinsèques auxquelles elles sont soumises, en maintenant un taux optimal de micronutriments dans les différents tissus, et ce en les apportant dans la microcirculation par un dispositif assurant leur passage par voie transdermique.
Par micronutriment on entend au sens de la présente invention des composés d'origine naturelle trouvés dans l'alimentation. Dans le cadre de l'invention, on utilise des micronutriments (ou microconstituants) qui ne sont pas indispensables pour le fonctionnement métabolique de l'organisme. Ils sont définis par les nutritionnistes comme les « microconstituants non indispensables » par A Martin dans « Les apports nutritionnels conseillés pour la population française » 3iéme ed 2001 TEC & DOC, pages 249-252 . Il s'agit de composés qui sont normalement apportés dans les aliments, mais pour lesquels il n'y a pas d'état carentiel. Le procédé selon l'invention n'a donc pas pour objectif de réparer une carence nutritionnelle avérée ni d'apporter au niveau de l'organisme un nutriment qui serait indispensable à son bon fonctionnement ou à sa survie.
Les micronutriments utiles dans l'invention sont des micronutriments ayant un effet sur la peau ou les phanères, notamment en améliorant leur aspect et/ou en renforçant leur résistance aux stress internes ou externes, comme par exemple des antioxydants.

Le brevet 6,475,514 décrit des patchs matriciels destinés aux athlètes en cours de compétition, contenant un mélange de nutriments indispensables tels que des carbohydrates, des électrolytes, des vitamines, du chrome et des minéraux; ils visent à compenser l'épuisement des réserves nutritionnelles et énergétiques survenant pendant un exercice physique intense.
L'administration transcutanée d'acides gras essentiels pour supplémenter des nourrissons déficients a été proposée par Lee et al (Archives of Disease in Childhood, 1993; 68: 27-28) ou Bougle et al (J. parenteral and enteral nutrition, 1986, 10, 216-219), mais ces auteurs concluent que cette voie d'administration est inefficace.
Par ailleurs, des systèmes de patchs ont déjà été proposés en cosmétique, mais il s'agit alors de dispositif destiné à assurer une meilleure pénétration de l'agent à activité au site d'action, comme par exemple dans la demande EP1002530 qui décrit des patchs anti-boutons contenant par exemple de l'acide salicylique.

Au contraire, le procédé de traitement cosmétique de l'invention permet, par l'application d'un dispositif transdermique, de distribuer dans la circulation, et donc dans des zones et des tissus du corps qui peuvent être au moins en partie distincts de la zone d'application du dispositif, un ou plusieurs micronutriments non indispensables au métabolisme, mais dont la présence contribue au maintien des qualités esthétiques de la peau ou des phanères.
La bio-disponibilité digestive des microconstituants non indispensables reste souvent un facteur limitant de leur efficacité en tant que compléments nutritionnels. Leur transfert, sous forme active, jusqu'aux couches cutanées superficielles par l'intermédiaire de la circulation sanguine est parfois difficile à obtenir (« e.g. Disposition and biotransformation of the estrogenic isoflavone daidzein in rats. » Bayer T, Colnot T, Dekant W Toxicol Sci 2001 Aug;62(2):205-11).
Les micronutriments ou microconstituants non indispensables selon l'invention peuvent être des molécules que l'organisme humain ou animal est capable de synthétiser ou des molécules que l'organisme n'est pas capable de synthétiser; dans ce second cas, il s'agit essentiellement de constituants apportés par les végétaux.

L'invention a également pour objet l'utilisation cosmétique d'au moins un micronutriment non indispensable dans un dispositif de délivrance transdermique, comme agent pour prévenir et/ou retarder et/ou limiter les signes du vieillissement d'un individu, en particulier les signes du vieillissement de régions de la peau, des muqueuses et/ou des phanères au moins partiellement distinctes de la zone d'application du dispositif de délivrance transdermique.
L'invention a également pour objet l'utilisation cosmétique d'au moins un micronutriment non indispensable dans un dispositif de délivrance transdermique, comme agent pour moduler la pigmentation de régions de la peau, des muqueuses et/ou des phanères au moins partiellement distinctes de la zone d'application du dispositif de délivrance transdermique.

Avantageusement, le micronutriment est présent dans la composition sous forme d'un extrait végétal ou animal ou d'un minéral le contenant.
Selon un mode de réalisation avantageux, la masse moléculaire du miconutriment adapté au procédé de l'invention est inférieure ou égale à 1000, notamment inférieure ou égale à 800, de préférence inférieure ou égale à 500.

De préférence, les micronutriments sont des composés amphiphiles, cette propriété pouvant être caractérisée par la mesure du coefficient de partage octanol/eau, noté P. Ce coefficient de partage P peut être déterminé par la méthode dite du "shake flask" telle que décrite dans "OCDE ligne directrice n° 107, Coefficient n-octanol / eau, méthode par agitation en flacon, 12 mai 1981 modifiée le 27 juillet 1995". Le coefficient de partage P représente le rapport à l'équilibre des concentrations du composé dans les deux phases non miscibles et constitue une mesure du caractère lipophile ou hydrophile de ce composé. L'affinité des molécules pour l'eau ou les graisses est exprimée en log P.
Les composés adaptés au procédé de l'invention seront avantageusement choisis tels que log P est supérieur ou égal à -2. Selon un autre aspect de l'invention, le coefficient de partage P des micronutriments entre l'octanol et l'eau est tel que log P est inférieur ou égal à 5.

Selon un des aspects de l'invention, le dispositif transdermique contient au moins un micronutriment choisi dans le groupe comprenant
* les polyphénols simples ou polymérisés tels que décrits par L Bravo dans Nutrition Reviews 1998 vol 56 N° 11pp 317 - 33, avec, les stilbènes , les lignanes et les flavonoïdes tels que décrits par GR Beecher dans J Nutr 2003 133 3248s-3254s avec les flavonols, les flavanols, les proanthocyanydines, les flavanones, les isoflavones les isoflavonoïdes,
* les caroténoïdes,
* le coenzyme Q10
* l'acide lipoique
* les acides aminés non indispensables comme par exemple la taurine
* leurs dérivés et analogues.

Les flavonoïdes sont formés d'un squelette 3-phénylchromane qui peut comporter des substituants variés et différents niveaux d'oxydation. Selon un mode préféré de réalisation de l'invention, les flavonoïdes utilisés sont des isoflavonoïdes qui constituent une sous-classe des flavonoïdes. Le terme isoflavonoïde regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les α-méthyldeoxybenzoines ou encore les 2-arylbenzofuranes. A cet égard, on se reportera avantageusement pour une revue complète sur les isoflavonoïdes, leurs méthodes d'analyse et leurs sources, au chapitre 5 "Isoflavonoïds" écrit par P.M. Dewick, dans The Flavonoïds, Harbone éditeur, pp. 125-157 (1988).
On préfère utiliser les isoflavonoïdes d'origine naturelle. Parmi ceux-ci, on peut citer : la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites, et leurs mélanges.

Les isoflavones sont préférées pour une utilisation dans la présente invention. Par ce terme, on entend aussi bien les formes aglycones (daidzéine, génistéine, glycitéine) que les formes glycosylées (daidzine, génistine, glycitine) des isoflavones.

Les catéchines sont des monomères pouvant présenter différentes formes isomères et constituent un sous groupe des flavonoïdes, qui comprennent également les flavanones, les flavones et anthocyanines, et les flavonols.
Le sous groupe des polyphénols catéchiques comprend un ensemble de composés classiquement isolés de plantes telles que le cacao, le thé, la vigne et ses dérivés, le pin (Pinus maritima), le cachou, certains fruits (Scalber A J. Nutr 2000 130 2073s-2085S ) et présentant un degré de polymérisation variable. L'unité de base, aussi appelée catéchine
ou catéchol est le penta hydroxy-3,5,7,3',4' dihydro-2,3 phényl-2 chromène, qui peut être sous forme cis ou trans; l'épicatéchine est son isomère, et peut également être présent sous forme cis ou trans. Les polyphénols catéchiques englobent aussi bien les divers isomères des unités de base (monomères), que des oligomères ( ou proanthocyanidols) ou des polymères (tannins).
Les catéchines utiles selon l'invention sont choisies dans le groupe comprenant: catéchine, épicatéchine, gallocatéchine, épigallocatéchine et leurs sels, leurs esters et/ou leurs dérivés, sous forme monomères. Par dérivés on entend notamment les molécules substituées par un plusieurs groupements méthyl ou éthyl.
On peut également utiliser des oligomères, ou proanthocyanidines; ils comportent avantageusement de 2 à 14 unités de base, notamment de 2 à 10; de préférence leur degré de polymérisation est inférieur ou égal à 5.

Les bases xanthiques utiles au sens de l'invention sont des dérivés de la xanthine, et comprennent notamment la théophylline, la caféine, la théobromine et les 1-hydroxyalkylxanthines et leurs sels compatibles tel que décrits par exemple dans FR-A-2 617401.

Les hydroxystilbènes sont des composés répondant à la formule générale 1 : 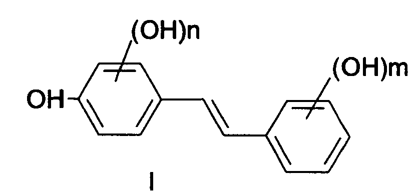
dans laquelle n est un nombre entier compris entre 1 et 4 inclusivement et m est un nombre entier compris entre 1 et 5 inclusivement. Ces composés peuvent être sous une forme Cis ou Trans.
Selon l'invention, le terme hydroxystilbène recouvre aussi bien les composés de formule I que leurs dérivés hydroxyalkylés.
Les stilbènes utilisables selon l'invention peuvent être ou non glycosylés. Les stilbènes et stilbènes glucosylés sont produits chez les plantes, essentiellement chez les spermatophytes, et font partie de la classe des molécules antibiotiques connues sous le nom de phytoalexines. Parmi celles-ci, un exemple bien documenté est le resvératrol ou 3, 4', 5-trihydroxystilbène.
Le resvératrol existe naturellement dans de nombreuses plantes et fruits sous forme simple (trihydroxystilbène) ou glucosylée (piceid, polydatine ou 4',5-dihydroxystilbène-3-O-β-mono-D-glucoside, par exemple). On trouve les deux formes, simple et glucosylée, en particulier dans la peau du raisin (Vrhovsek *et al.,* Am. J. Enol. Vitic., vol. 48, n° 2, 1997) ou encore dans des surnageants de cultures *in vitro* de *vitis vinifera* (Teguo *et al.,* J. Nat. Prod., 61, 655-657, 1998).
Les propriétés des stilbènes et hydroxystilbènes ont été mises à profit dans la réalisation de compositions cosmétiques contenant ces composés. Il est décrit par exemple des compositions cosmétiques comprenant du resvératrol et leurs utilisations pour lutter contre les signes du vieillissement cutané, pour lisser la peau ou pour traiter les rides et les ridules (WO 99/04747, Unilever, N.V.). Il est aussi décrit un procédé d'obtention de dérivés esters de resvératrol et leurs utilisations dans des compositions cosmétiques en tant que précurseur de resvératrol (WO 99/03816, Caudalie). Cependant, à la connaissance des inventeurs, il n'a jamais été proposé de les appliquer sous forme d'un dispositif de délivrance transdermique en vue de limiter l'apparition des signes du vieillissement de la peau et/ou des phanères, ni en vue de moduler la pigmentation de la peau et/ou des phanères.

Parmi les hydroxystilbènes, on peut citer les mono, di, tri, tétra, penta, hexa, hepta, octo, nonahydroxystilbènes, ou encore leurs dérivés hydroxyalkylés.

Selon l'invention les hydroxystilbènes peuvent être utilisés seuls ou en mélanges de toute nature, sous forme glycosylée en particulier glucosylée tels que décrits dans EP1175888 ou non glucosylée, et peuvent être d'origine naturelle ou synthétique.

Les hydroxystilbènes utilisables selon l'invention sont notamment choisis parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

Préférentiellement, on utilise selon l'invention du 3,4',5-trihydroxystilbène ou resvératrol.

Selon l'un des modes de réalisation de l'invention, le micronutriment non indispensable peut être associé à un micronutriment indispensable, notamment un microconstituant susceptible de renforcer son activité par un mécanisme de type synergique ou en améliorant sa pénétration. La composition du dispositif transdermique peut donc contenir en outre des minéraux et vitamines connus pour être importants pour la peau et les phanères, tels que publiés par Boelsma E, van de Vijver LP, Goldbohm RA, Klopping-Ketelaars IA, Hendriks HF, Roza L (« Human skin condition and its associations with nutrient concentrations in serum and diet. » Am J Clin Nutr. 2003 Feb;77(2):348-55). Des vitamines pourront ainsi être présentes dans les compositions des systèmes de délivrance transdermqiue selon l'invention. Il peut s'agir de vitamines principalement liposolubles, comme les vitamines A, notamment rétinol, acétate de rétinol, palmitate de rétinol, bêta-carotène, les vitamines D, en particulier D2, D3, le cholécalciférol et l'ergocalciférol, ou la vitamine E qui englobe des composés tocophéryle comme le D-alpha-tocophérol, le DL- alpha-tocophérol, l'acétate de D-alpha-tocophérol, l'acétate de DL- alpha-tocophérol et le succinate de D- alpha-tocophérol.
On peut également utiliser des vitamines hydrosolubles; parmi celles-ci on peut citer les vitamines B, notamment la vitamine B1 sous forme par exemple de chlorhydrate de thiamine ou de mononitrate de thiamine, la vitamine B2 sous forme par exemple de riboflavine ou riboflavine-5'-phosphate de sodium, la vitamine B6 sous forme par exemple de chlorhydrate de pyridoxine ou de pyridoxine-5'-phosphate, la vitamine B8 sous forme par exemple de biotine, la vitamine B12 sous forme par exemple de cyanocobalamine ou d'hydroxycobalamine, mais aussi la vitamine PP ou niacine sous forme par exemple de nicotinamide adénine dinucléotide (NAD) ou nicotinamide adénine dinucléotide phosphate NADP ou d'acide nicitinique ou de nicotinamide ou de nyacitine, ou la vitamine C sous forme par exemple d'acide L-ascorbique, de L-ascorbate de sodium, L-ascorbate de calcium, L-ascorbate de potassium ou L-ascorbyl 6-palmitate.
Dans un mode de réalisation avantageux, le système de délivrance transdermique ne contient pas de complexe de peptide et de cuivre, en particulier de tripeptide de cuivre tel que décrit dans WO2004/014413.

De préférence, le micronutriment non indispensable sera choisi parmi,
- les bases xanthiques naturelles comme la caféine, la théobromine,
- les polyphénols en particulier les catéchines, la théaflavine, la nobilétine, le resvératrol, le resvératrol glucosylé, la génistéine, la daidzéine, la daidzine, la génistine, le β carotène, le lycopéne, la lutéine, la zéaxanthine, l'apigénine et leurs mélanges en toutes proportions; pour une action plus spécifique sur la pigmentation on peut citer également la silibinine, les procyanidines, le resvératrol, le resvératrol glucosylé.
- L'acide lipoïque, la taurine, les caroténoïdes sont également préférés pour moduler la pigmentation.

Les théaflavines sont des flavanols du thé noir et du thé oolong qui possède en particulier une structure benzotropolone la structure générale est décrite par GR Beecher dans J Nutr 2003 133 3248s-3254s. La nobilétine est un polymethoxyflavone (3',4',5,6,7,8-hexamethoxyflavone) isolé des fruits Citrus depressa

Les micronutriments adaptés à la mise en oeuvre de l'invention devront être compatibles avec les formulations des dispositifs d'administration transdermique. Selon l'un des modes de réalisation de l'invention, ces micronutriments présenteront une bonne stabilité physico-chimique, c'est à dire ne présentent pas de dégradation de leur structure et/ou de leurs propriétés notamment après une exposition à l'air ou à la lumière modérée. Cependant, selon un autre mode de réalisation, les micronutriments adaptés ne présentent pas nécessairement une bonne stabilité physico chimique. Par exemple, les caroténoïdes et certaines vitamines sensibles à la lumière qui ne pouvaient être appliquées par voie topique sur les zones exposées au soleil, pourront avantageusement être mis en oeuvre selon l'invention. De préférence, les micronutriments actifs ne seront pas ou peu métabolisés au niveau cutané, mais on pourra utiliser une forme précurseur qui sera métabolisée sous la forme active au moment de la pénétration ou dans la circulation.

Selon l'un des aspects de l'invention, le micronutriment sera présent dans la composition du dispositif transdermique sous forme purifiée, qu'il soit d'origine naturelle et obtenu par extraction, généralement à partir d'une plante, ou d'origine synthétique.
Selon un autre aspect, le ou les micronutriments présents dans la composition seront sous forme d'un extrait naturel, en particulier un extrait végétal ou d'algues, le contenant.
Des extraits adaptés à la mise en oeuvre de l'invention sont des extraits titrés en principe actif comme par exemple des extraits de végétaux du genre Camellia (autrement également appelé Theaceae); ce genre comporte au moins 80 espèces parmi lesquelles on peut citer les espèces Camellia cuspidata, Camellia japonica, Camellia sasanqua, Camellia reticulata, Camellia saluensis ou encore Camellia sinensis. On utilisera notamment des extraits de thé vert, à teneur garantie en catéchines et antioxydants, mais aussi des extraits de thé noir ou blanc, des extraits d'hibiscus aussi appelé thé rose; de manière non limitative, on peut citer aussi des extraits de Vitis vinifera et notamment des extraits de pépin de raisin titrés en polyphénols, des extraits de fruits comme par exemple des extraits de cerise, citron, orange, melon, pastèque, pomme, banane, kiwi, des extraits de cacao, notamment de coque de fève de cacao, des extraits de soja, des extraits de pâte de tomate, des extraits d'ail, oignon, courge, aubergine, épinards, rhubarbe et de fleurs comestibles (rose, pensée, géranium), des tisanes et des thés alimentaires, des huiles alimentaires, des extraits de plante choisies parmi le tilleul, la camomille, le chrysantème, la fleur de courgette et la fleur d'oranger, le romarin, la myrtille, la mûre (Morinda citrifolia L.).
Par exemple, on utilise un plante du genre Vaccinium. On broie des fruits secs et on fait une extraction éthanolique (95% Ethanol) :obtention d'une solution colorée. On pourra titrer en certains anthocyannes malvidin 3-galactoside, delphinidin 3-galactoside, delphinidin 3-arabinoside, petunidin 3-galactoside, petunidin 3-arabinoside, et malvidin 3-arabinoside. L'éthanol est ensuite évaporé pour obtenir la poudre à formuler en patch.
Comme extrait de romarin, on utilise un plante du genre Rosmarinus. On broie la plante entière (ou certaines parties feuilles, tiges racines ou cellules indifférentiées) dans l'eau à température ambiante. Les particules solides restantes sont éliminées (décantation, centrifugation, filtration). La solution est stérilisée par ultrafiltration à 0.22 µm. Une autre méthode consiste à stériliser en portant à ébullition le broyat de plante puis à éliminer les particules solides. On peut ensuite lyophiliser.
Comme extrait de mûre on peut utiliser un extrait obtenu à partir d'une plante polynésienne Tahitian Noni(R)" acheté chez Morinda, Inc.. On broie des fruits secs et on fait une extraction éthanolique (95% Ethanol) :obtention d'une solution colorée. L'éthanol est ensuite évaporé pour obtenir la poudre à formuler en patch.
On peut également selon l'invention utiliser un extrait végétal enrichi en polyphénol comme ceux préparés à partir de germes de légume, d'haricot mung (Vigna radiata) par Kalidas Shetty & al. Ce type de préparation à partir d'aliment naturel par micro-onde, on enrichit en fraction polyphénolique et stérilise sur le plan bactérien l'extrait qui devient applicable de fait sur la peau; on filtre à 0.22 µm la soupe obtenue, on déshydrate (lyophilisation) pour mettre la poudre dans un patch.

On peut notamment utiliser des extraits alcooliques, hydroalcooliques ou organiques, des extraits aqueux. Les extraits totaux sont obtenus par broyage du végétal ou de la partie de végétal concerné selon le type de polyphénol dont on veut enrichir l'extrait, comme la peau d'orange pour obtenir la nobilétine, puis récupération de l'ensemble du broyat, éventuellement suivi de l'élimination partielle des grosses particules insolubles.

Des extraits particulièrement adaptés à la mise en oeuvre de l'invention sont notamment des extraits de myrtille, des extraits de romarin et des extraits de thé, vert ou noir.

L'homme du métier adaptera les quantités des micronutriments ou des extraits alimentaires les contenant en fonction de l'effet recherché, qui pourront varier notamment de 0,001 à 30% par rapport au poids de la composition. A titre indicatif, on pourra utiliser une poudre de cacao, ou une pâte de tomate commerciale à des concentrations de 0,1 à 10% par rapport au poids total de la composition. Dans le cas d'un extrait de romarin, les concentrations couramment utilisées seront généralement inférieures ou égales à 20%, mais supérieures ou égales à 0,1%, par rapport au poids total de la composition.
L'utilisation d'extraits végétaux est particulièrement avantageuse puisqu'elle évite l'administration de molécule chimique mais implique uniquement des composés naturels, en particulier des extraits naturels alimentaires végétaux, dont on peut parfaitement garantir la sécurité du fait de leur ingestion quotidienne. C'est souvent la combinaison d'un mélange de composés nutritionnels qui apporte le meilleur résultat Ceci est rendu possible par ce mode d'administration qui assure une bonne diffusion dans la circulation générale et permet aux actifs d'atteindre le derme et les cellules cibles sans subir de dégradation par les enzymes digestives.
Le procédé selon l'invention permet ainsi d'obtenir des taux de substances actives suffisant pour observer un effet sans recourir à des doses importantes de produit; de plus, les concentrations sanguines sont maintenues grâce à la diffusion progressive des micronutriments libérés à partir du dispositif transdermique, ce dernier pouvant rester en place plusieurs heures, voire plusieurs jours.
Avantageusement, le dispositif transdermique comprendra une association des micronutriments tels que définis précédemment, en particulier au moins deux micronutriments, notamment au moins deux micronutriments non indispensables.
Le procédé de traitement cosmétique selon l'invention sera particulièrement un procédé destiné à prévenir des modifications indésirables de l'aspect esthétique de la peau ou des phanères, en obtenant conjointement un effet général nutritif et un effet local, par le maintien de taux physiologiques des nutriments pouvant avoir un effet sur la peau ou les phanères.

Selon encore un autre aspect de l'invention, le procédé de traitement cosmétique permet de traiter de manière préventive et/ou curative, les signes cutanés du vieillissement qu'il soit chronobiologique ou photo-induit, particulièrement l'amincissement du derme et/ou la dégradation des fibres de collagène. Le procédé permet aussi de prévenir ou de diminuer la perte de fermeté de la peau liée au vieillissement plus particulièrement à traiter, de manière préventive ou curative, la peau flasque et/ou ridée, et/ou à stimuler le raffermissement de la peau.

Par exemple, à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des rides et ridules de surface. La peau présente un aspect rugueux à la palpation. Enfin la peau présente une souplesse diminuée.
Par signes cutanés du vieillissement on entend toutes modifications de l'aspect extérieur de la peau dues au vieillissement qu'il soit intrinsèque ou extrinsèque; on entend ainsi à la fois le vieillissement chronobiologique et/ou le vieillissement induit (vieillissement photoinduit, vieillissement du aux agressions oxydatives induites par l'environnement, le tabagisme ou le stress), comme par exemple les rides et ridules, la peau flétrie, la peau molle, la peau amincie, la peau terne et sans éclat, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement des fibres de collagène, consécutives à une exposition aux rayonnements ultra-violets, ou la glycation des protéines, qui peuvent avoir comme conséquence l'amincissement et une diminution de la souplesse du derme.
Le procédé cosmétique peut selon l'un des aspects de l'invention être destiné à favoriser le lissage de la peau et/ou à tendre la peau .

Les micronutriments adaptés à la mise en oeuvre de ce procédé sont notamment les isoflavones comme la génistéine ou la daidzéine, ou les composés ayant une activité anti-radicalaire comme les catéchines.
Parmi les signes du vieillissement chronobiologique ou photo-induit, on peut également citer les altérations de la pigmentation de la peau ou des phanères. Sur la peau, on peut voir apparaître des taches pigmentaires, ou des lentigos qui nuisent à l'aspect et à l'esthétique de l'individu.
Selon un autre aspect de l'invention, le procédé de traitement cosmétique visera à améliorer ou à favoriser la pigmentation de la peau ou des phanères, notamment des cils et/ou des cheveux. Le procédé peut ainsi être destiné à lutter contre le blanchiment des poils et des cheveux, et à diminuer ou retarder l'apparition des cheveux blancs, aussi appelée canitie.
Le procédé peut aussi être destiné à lutter contre la réduction de la densité et/ou du diamètre des cheveux qui apparaît avec l'age chez l'Homme et la Femme.

Des micronutriments adaptés à ce mode de réalisation sont notamment la taurine ou l'acide lipoïque.
Avantageusement, le dispositif transdermique comprend des micronutriments choisis parmi les catéchines et les bases xanthiques citées plus haut.

Le procédé de traitement cosmétique pourra moduler la pigmentation de la peau en améliorant sa résistance aux effets des rayonnements UV, et notamment en favorisant la synthèse de molécules biologiques impliquées dans les mécanisme de protection et de réparation des stress induits par l'exposition au rayonnement solaire. Les micronutriments présents dans le dispositif transdermique de l'invention seront alors plus particulièrement l'apigénine la silibinine, la lutéine et/ou l'acide lipoïque.

Selon un autre mode de réalisation, le procédé selon l'invention vise à limiter, prévenir ou diminuer la pigmentation de la peau et/ou des phanères et/ou à éclaircir le teint, et notamment à atténuer les taches pigmentaires.
Des micronutriments adaptés à ce mode de réalisation sont notamment les hydroxystilbènes tels que le resvératrol et ses dérivés, ou les polymères catéchiques de type procyanidine ou tannins.

Le procédé sera également adapté pour moduler la pigmentation de la peau photoinduite et/ou favoriser la résistance de la peau aux rayonnements U.V.

Le procédé de traitement cosmétique de l'invention sera appliqué de manière préventive
ou curative, à un mammifère en particulier un être humain dont l'équilibre nutritionnel risque d'être perturbé, notamment au cours de certaines époques physiologiques de la vie, lors de modifications du rythme de vie entraînant notamment un stress ou de la fatigue, en prévision d'une exposition à la pollution ou aux rayonnements ultra-violets.
Le dispositif transdermique sera appliqué conformément à l'invention sur une zone du revêtement cutané qui n'est pas, au moins en partie, identique à la région où les effets sont recherchés. Cela constitue une différence essentielle avec les procédés mettant en oeuvre des systèmes de délivrance transdermique utilisés jusqu'à présent, qui visent uniquement une activité au point d'application.
Le dispositif transdermique sera ainsi appliqué sur toute zone présentant une vascularisation suffisante, notamment sur des parties du visage, derrière l'oreille, sur l'épaule, sur la région abdominale ou sur les bras. Il pourra être maintenu en place entre 30 minutes et plusieurs jours, notamment de une à huit heures: on peut par exemple le laisser en place une nuit, ou bien pendant 24heures. L'application sera renouvelée, en laissant éventuellement une fenêtre sans application de dispositif transdermique. Le procédé sera bien entendu adapté par l'homme du métier en fonction des composés actifs présents dans la composition, de leur vitesse de diffusion et du type de système de délivrance transdermique utilisé, ainsi que des taux plasmatiques correspondant à une activité optimale dans les zones corporelles visées.

La taille et la forme du dispositif ou système de délivrance transdermique utile selon l'invention seront adaptés par l'homme du métier mais à titre indicatif, le dispositif, solide ou semi-solide, aura généralement une surface inférieure ou égale à 25 cm², notamment de 0,25 cm² à 20 cm², de préférence supérieure ou égale à 3 cm²; des dispositifs adaptés à l'invention pourront par exemple avoir une surface de 5 à 15 cm².

L'invention a également pour objet l'utilisation d'au moins un micronutriment tel que défini dans ce qui précède, pour la préparation d'un dispositif de délivrance transdermique, ou d'une composition pouvant être intégrée dans un dispositif transdermique destiné à prévenir et/ou retarder les altérations de la peau, du cuir chevelu et/ou des phanères.
Les compositions sont à usage cosmétique ou dermatologique.

Par 'dispositif transdermique', ou système de délivrance transdermique au sens de l'invention on entend tout système permettant une libération active ou passive de la substance active par transdermie, c'est à dire permettant son transfert à travers la peau jusqu'à la circulation générale systémique. Parmi les dispositifs transdermiques applicables à l'invention, on peut citer par exemple :
- des patchs, c'est-à-dire tout système de délivrance d'actif présentant une structure composite sous forme de couches qui, par application sur la peau, assure la libération du produit actif par transdermie. On peut citer par exemple :
   - des patchs à libération contrôlée avec une matrice polymérique hydrophobe tels que décrits par exemple dans FR 2 738 744 ;
   - des patchs de type réservoir contenant un réservoir de substance active, une membrane de diffusion et une couche adhésive
   - des patchs à effet de champ magnétique favorisant la pénétration des actifs dans la peau tels que décrits par exemple dans FR 2 791 750 ;
   - des patchs à adhérence optimisée, comportant une couche de polymère hydrophobe fixée sur une couche support et contenant des particules de composé actif, des particules d'huile et des particules d'un agent hydro-absorbant tels que décrits par exemple dans FR2 761 889 ;
   - des gels patchs à forte teneur en eau, de type hydrogel, notamment à base d'au moins un hydrocolloïde et/ou de polysaccharides, de protéines, de polyacrylamide, d'acide polyacrylique, de copolymères d'acide acrylique et d'amidon, de polyvinylpyrrolidone ou de dérivés d'acide polyvinylique, et présentant une adhésion de contact, similaire à celle résultant d'un effet ventouse.
   - Des gels patchs hydrophobes, aussi appelés organogels ou oléogels à base de polyéthylène, de silicones ou de copolymère de styrène et d'isoprène ou de copolymères de styrène et butadiène. Ces systèmes ont l'avantage de pouvoir contenir de fortes proportions de composés hydrophobes
   - Des gels patchs à base de de polyuréthanes segmentés (SPUG) dont le caractère lipophile ou hydrophile peut être modifié par modification des chaînes polyoxyéthylénées (Y. Shikinami, Adhesive polymer gels for medical uses, Kagaku To Tokyo, 67 (4), 141-150, 1993)
- des feuilles ou pastilles de types pastilles anti-rides décrites dans FR 2 512 651 ou FR 2 538 247 ;
- des films composites tels que décrits dans EP-A- 0 285 563 ; avantageusement, on pourra utiliser un film composite comportant une couche réservoir constituée par une matrice formée d'un polymère de silicone à l'intérieur de laquelle sont disposées des inclusions constituées par la phase aqueuse active gélifiée grâce à au moins un agent gélifiant (FR 2 650 747 L'Oreal) ;
- De films fins solides à base de polymères naturels ou synthétiques qui appliqués sur la peau préalablement mouillée se dissolvent in situ; de tels films sont décrits dans les demande de brevet WO 02/053197, WO 02/05789
- Des films contenant un agent filmogène et un polymère adhésif hydrophile, aussi appelés, patch-non-patch, tels que décrits dans le brevet WO 02/30402
- Des films obtenus à partir de pulvérisation de poudres filmogènes, tels que décrits dans FR0115069,
- des compositions à base de polymères ayant des propriétés adhésives capables de former un film que l'on retire après séchage (EP- 0 514 760, EP-0 826 364) ou une couche solide élastique et pliable retirée en appliquant un tissu ou un plastique adhésif (WO93/05893) ou des gels ou pâtes ('masques de beauté') qui, après application sur le visage, sèchent pour donner un film que l'on retire par lavage, nettoyage ou arrachage ; on parlera généralement de 'vemis-patch' ou 'peel-patch'.

Mais on entend également tous les systèmes augmentant l'absorption percutanée par un mécanisme actif (M.R. Prausnitz et al., Current status and future potential of transdermal drug delivery, Nature Rev., vol. 3, 115-124, 2004) :
- Systèmes d'injection sous pression ou sans-aiguille (Burkoth T.L. et al., *Transdermal and transmucosal powdered drug delivery.,* Crit Rev Ther Drug Carrier Syst. 1999;16(4):331-84
- Utilisation d'ultra-sons : Sonophorèse et phonophorèse (Joshi A. and Raje J., *Sonicated transdermal drug transport,* J. Control. Release, 83 (1), 13-22, 2002)
- Utilisation de courants électriques : lontophorèse, electroporation, courant microfréquence (Rivière J.E. and Heit M.C., *Electrically-assisted transdermal drug delivery,* Pharm. Res. 14 (6), 687-697, 1997) (brevets US 6,148,232, WO 03/035167)
- Utilisation d'ondes magnétiques : magnétophorèse (Murthy S.N., *Magnetophoresis: an approach to enhance transdermal drug diffusion.,* Pharmazie. 1999 May;54(5):377-9 )
- Utilisation de micro-aiguilles ( Mc Allister D.V. et al., *Microfabricated microneedles for gene and drug delivery,* Annu. Rev. Biomed. Eng., 2, 289-313, 2000) (brevet US 6,451,240)

Ces systèmes de délivrance transdermique seront formulés selon des techniques connues de l'homme du métier pour assurer le taux et la vitesse de diffusion adaptée aux micronutriments présents dans la composition.
De préférence, les systèmes de délivrance transdermique utiles pour la mise en oeuvre de l'invention sont des systèmes, et plus particulièrement des patchs, favorisant le transport actif des molécules, par opposition aux systèmes ne reposant que sur la diffusion passive des molécules.

En particulier, ils pourront contenir des accélérateurs de pénétration, notamment choisis parmi les mono- et di-alcools non cycliques, l'acétate d'éthyle, l'acétate de butyle, le myristate d'isopropyle, les acides gras, les phospholipides, les terpènes, l'azone et dérivés, le propylène glycol et dérivés glycoliques, les cyclodextrines, l'octylsalicylate, le cyclopentadecanolide, les polysorbates, la polyvinylpyrrolidone et dérivés, cette liste n'étant pas limitative.
D'autres systèmes de délivrance transdermique particulièrement adaptés à l'invention sont les patchs à effet de champ magnétique ou des systèmes associant un patch ou tout autre dispositif décrit plus haut, à un moyen pour augmenter le transport du ou des micronutriments à travers l'épiderme pour atteindre la microcirculation; de tels moyens sont choisis en particulier dans le groupe suivant:
- Systèmes d'injection sous pression ou sans-aiguille (Burkoth T.L. et al., *Transdermal and transmucosal powdered drug delivery.,* Crit Rev Ther Drug Carrier Syst. 1999;16(4):331-84; Il s'agit de dispositifs capables de faire pénétrer dans la peau de fines particules accélérées sous un flux de gaz sous pression. De tels systèmes sont proposés par des sociétés telles que BIOJECT, POWDERJECT ou MEDIJECT.
- Utilisation d'ultra-sons : Sonophorèse et phonophorèse (Joshi A. and Raje J., *Sonicated transdermal drug transport,* J. Control. Release, 83 (1), 13-22, 2002). Utilisent des ultrasons de basse fréquence pour augmenter de façon transitoire la perméabilité cutanée (US 6234990, US 6190315). Un produit, le SonoPrepTM, a notamment été développé par la société SONTRA.
- Utilisation de courants électriques : lontophorèse, electroporation, courant microfréquence (Riviere J.E. and Heit M.C., *Electrically-assisted transdermal drug delivery,* Pharm. Res. 14 (6), 687-697, 1997) (brevets US 6,148,232, WO 03/035167). Ces techniques ont en commun de générer un courant électrique qui va augmenter le passage transdermique d'entités chimiques chargées ou neutres. L'iontophorèse pourra être réalisée avec un courant de faible intensité (< 2 mA) avec des systèmes existants tels que l'IontopatchTM proposé par la société TRAVANTI PHARMA.
- Utilisation d'ondes magnétiques : magnétophorèse (Murthy S.N., *Magnetophoresis: an approach to enhance transdermal drug diffusion.,* Pharmazie. 1999 May;54(5):377-9 )
- Utilisation de micro-aiguilles ( Mc Allister D.V. et al., *Microfabricated microneedles for gene and drug delivery,* Annu. Rev. Biomed. Eng., 2, 289-313, 2000) (brevet US 6,451,240). Les micro-aiguilles permettent de créer réversiblement dans la barrière cutanée des micro trous à travers lesquels les ingrédients actifs vont pénétrer de façon optimale. Les micro-aiguilles seront choisies préférentiellement d'une taille inférieure à 200 µ et seront constituées de silicium ou de sucres.

Selon un mode de réalisation particulier de l'invention, le transport actif du ou des micronutriments sera favorisé par au moins une étape de prétraitement permettant de préparer la peau à l'activité du système de délivrance transdermique. Cette étape de prétraitement pourra consister à appliquer un traitement choisi parmi le peeling, la dermabrasion et/ou le dégraissage de la peau. On pourra aussi effectuer de façon successive ces différents types de traitement, sur la zone du corps où le système transdermique sera ensuite appliqué.
Le peeling sera un peeling par action chimique ou mécanique. Il peut ainsi être effectué par application d'une composition de peeling comportant un au moins un agent desquamant. De tels agents sont connus de l'homme du métier, il s'agit notamment des α-hydroxyacides, comme l'acide lactique ou l'acide glycolique, des β-hydroxyacides, de l'urée ou de l'HEPES. Les concentrations seront adaptées par l'homme du métier selon les actifs et le type de système transdermique utilisé dans les étapes ultérieures.
Un peeling superficiel peut être réalisé par pulvérisation sous haute vélocité de nanogoutelettes d'eau ou d'une solution aqueuse mélangées ou non à un gaz, tel que l'oxygène. De tels systèmes sont disponibles commercialement, comme par exemple le JET-PEEL ( société Tav-Tech) ou le WATER BEAM (société Medicamat).

La dermabrasion, ou microdermabrasion, est une exfoliation qui peut notamment être réalisée par application d'une composition contenant des particules d'oxyde métallique, en particulier d'un oxyde de magnésium ou d'aluminium, qui est appliquée par massage sur la peau, de préférence préalablement mouillée, puis éliminée par lavage.

Le dégraissage de la peau sera réalisé par application de produits détergents doux qui élimineront ou diminueront le sébum naturellement présent à sa surface.

L'un des objets de l'invention est donc un procédé de traitement cosmétique pour prévenir et/ou retarder les signes du vieillissement de la peau, des muqueuses et/ou des phanères, comprenant (i) au moins une première étape de prétraitement d'une zone de la peau ou du cuir chevelu par peeling, microdermabrasion et/ou dégraissage, et (ii) une étape ultérieure dans laquelle on applique sur ladite zone de peau et/ou de cuir chevelu soumise au prétraitement un système de délivrance transdermique, la composition du système transdermique contenant au moins un micronutriment non indispensable tel que défini précédemment.

Les systèmes de délivrance transdermique contiendront les excipients adaptés à leur formulation connus de l'homme du métier et notamment au moins un composé choisi parmi les gélifiants, les polymères adhésifs, les stabilisants, les colorants, les pigments, les charges, les solvants, les ions...ils pourront également contenir des agents apaisants, en particulier dans le cas où un prétraitement de la peau est effectué avant leur application.

La matrice adhésive peut notamment comporter un ou des polymères naturels ou synthétiques hydrophiles capables de gélifier en présence d'eau et de présenter un pouvoir adhésif. Ces polymères hydrophiles seront préférentiellement choisis parmi l'acide acrylique, ses dérivés et co-polymères et seront présents dans une proportion comprise entre 5 et 60% de la matrice adhésive, de préférence entre 10 et 40%. La matrice adhésive peut, dans certains modes de réalisation, contenir un ou plusieurs autres hydrocolloïdes.
Le ou les hydrocolloïdes utilisés, peuvent être par exemple choisis dans le groupe formé par:
- la gomme de gellane ;
- la cellulose ou ses dérivés comme la carboxyméthylcellulose, l'hydroxypropylcellulose, la méthylcellulose, l'hydroxypropylméthylcellulose ou l'hydroxyéthylcellulose ainsi que les celluloses modifiées notamment par greffage de groupement alkyle ;
- les extraits d'algue tels que l'agar-agar, les carraghénanes, les alginates ;
- les extraits de graines tels que la gomme de caroube, la gomme de guar, les gommes de guar modifiées notamment par greffage de groupement alkyle ;
- les exsudats de plantes tels que la gomme arabique, la gomme karaya, la gomme adragante, la gomme de gatty ;
- les exsudats de micro-organismes tels que la gomme de xanthane ;
- les extraits de fruits tels que les pectines ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT,
- les produits du type POLYCARE®, commercialisé par la société Rhone-Poulenc.

ou un mélange de ces composés.

Ces agents seront choisis selon leurs propriétés propres afin de moduler l'adhésion et la viscosité de la matrice. Cette dernière pourra en outre renfermer des agents hydratants, tels que le glycerol, dans une proportion de 0.1 à 30%, préférentiellement de 1 à 20%. En outre, la matrice adhésive pourra comprendre un ou des excipients choisis parmi les conservateurs, les colorants, les parfums, les stabilisants, les initiateurs de polymérisation. La matrice adhésive peut être laminée sur un support plus ou moins souple, non soluble consistant en un support tissé ou non-tissé ou encore un support en matériau plastique choisi préférentiellement parmi le polyuréthane (commercialisé par exemple sous les marques BAYDUR®, DALTOFLEX®, UROFLEX®, HYPERLAST®), polyuréthane élastomère thermoplastique (DESMOPAN®, ESTANE®, LASTANE®, TEXIN®), élastomère thermoplastique SBS (styrène-butadiène-styrène) (CARIFLEX®, KRATON ®, SOLPRENE®), élastomère thermoplastique SEBS (Styrène-ethylène-butylène-styrène) (KRATON®), EVA (Ethylène-Vinyl acétate) (ELVAX®, ESCORENE®, OPTENE®), élastomère thermoplastique coether ester (CEE TPE) (ARNITEL®, HYTREL®, RITEFLEX®), polyéthylenes, polypropylènes, silicones.
La structure du support non soluble peut soit être respirante, soit être généralement occlusive, c'est-à-dire constituée d'une matière imperméable à l'air et/ou la vapeur d'eau, de façon à faciliter la pénétration de l'actif dans l'épiderme.

La matrice adhésive peut comporter en outre un ou plusieurs actifs choisis parmi la liste suivante : laponite, tensioactifs, collagène, acide salicylique, huiles essentielles aromatiques, colorants, anti-oxydants, anti-radicaux libres, hydratants, dépigmentants, liporégulateurs, anti-acnéiques, anti-séborrhéiques, anti-vieillissement, adoucissants, anti-rides, kératolitiques, anti-inflammatoires, rafraîchissants, cicatrisants, protecteurs vasculaires, anti-bactériens, anti-fongiques, anti-perspirants, déodorants, conditionneurs de la peau, insensibilisants, immunomodulateurs et nourrissants.

Ainsi, le système de délivrance d'actif selon l'invention peut être un patch de structure classique comportant plusieurs couches successives dans l'ordre suivant : une première couche, formée du support non soluble ; une seconde couche, dite matrice adhésive, fixée sur la couche support et contenant le micronutriment, cette couche venant directement au contact de la peau; enfin, éventuellement, une couche pelable de protection, recouvrant de façon hermétique la couche de polymère, de façon à la protéger de toute contamination extérieure pendant le temps de stockage préalable à l'utilisation du patch.

La couche support a non seulement pour fonction de supporter la couche de polymère, mais également de servir de revêtement protecteur de celle-ci. Elle peut être de même dimension que la couche de polymère ou s'étendre au-delà de la couche de polymère et vers l'extérieur, de manière à pouvoir éventuellement recevoir des moyens adhésifs disposés à la périphérie de la couche de polymère.

Dans une autre version de l'invention, la matrice adhésive sera composée de polymères synthétiques amphiphiles comme par exemple les copolymères acrylique/vinylique, méthacrylique/vinylique, les acrylamides substituées, les copolymères de styrène et d'isoprène, les copolymères de styrène et butadiène, les polyuréthanes segmentés ou encore les polydiméthylsiloxanes (BIO-PSA® de la société DOW-CORNING). Le ou les micronutriments sont incorporés directement dans la matrice adhésive. La composition exacte de la matrice adhésive et le choix du ou des polymères seront fonction des propriétés physico-chimiques du ou des micronutriments ainsi que de la cinétique de diffusion recherchée.
A titre indicatif, la composition du patch pourra comprendre par exemple des copolymères éthylène vinylacétate à des concentrations variant de 50 à 80%, un polyisobutylène adhésif à des concentrations variant de 1 à 10%, du polyacrylate de sodium à des concentrations de 5 à 15%, de 5 à 10% d'acide polyacrylique, de 1 à 2% d'acide tartarique, de 2 à 3% de Tween 80, de 5 à 10% de carboxyméthyl cellulose et/ou de 10 à 30% de glycérine; l'ensemble de ces pourcentages est exprimé par rapport au poids total de la composition et inclut les bornes de l'intervalle mentionné.

Le dispositif de délivrance transdermique adapté à la mise en oeuvre du procédé selon l'invention pourra notamment être un patch choisi parmi les patchs à libération contrôlée, les patchs réservoirs, les patchs à effet de champ magnétique, les gels patchs à base d'hydrocolloïde, les gels patchs huileux et les films composites de type réservoir; le dispositif peut ainsi contenir des extraits de Vitis vinifera titrés en resvératrol et en resvératrol glucosylé, à une concentration comprise entre 0,001 et 10 g/cm³ de composition, et/ou des extraits de thé vert titré en épigallocatéchine gallate, de thé noir ou oolong titrés en théaflavine pour une concentration finale en ces micronutriments de 0,001 à 10 g/cm³.
Des micronutriments particulièrement adaptés à la mise en oeuvre de l'invention sont des extraits de myrtille et ou des extraits de thé titrés en catéchine.

L'invention a également pour objet l'utilisation cosmétique d'au moins un micronutriment non indispensable dans un dispositif transdermique comme agent pour diminuer et/ou retarder les signes du vieillissement d'une zone de la peau et/ou des muqueuses et/ou des phanères au moins partiellement distincte de celle où le dispositif de délivrance transdermique est appliqué.

D'autres avantages de l'invention apparaîtront à la lecture des exemples qui suivent.

### Exemple 1: formulations

| | |
|---|---|
| poudre de cacao** | 4% |
| Pâte de Tomate * | 3% |
| DURO-TAK 36-6172*** | 82 % |
| Diméthicone | 1% |

| | |
|---|---|
| *Tomato paste Heinz® | |
| **Van Houten® | |
| *** adhésif sensible à la pression | |

### Exemple 2

| | |
|---|---|
| Extrait de romarin* | 6% |
| Sodium polyacrylate | 5% |
| Polyacrylic acid | 5% |
| Tartaric acid | 2% |
| Tween 80 | 3% |
| Glycerine | 30% |
| Méthylparaben | 0,5% |
| Propylparaben | 0,5% |
| Eau qsp | 100% |

| | |
|---|---|
| * obtenu par broyage de la plante entière dans l'eau à température ambiante, élimination des particules solides restantes et stérilisation par ultrafiltration à 0,22µm. | |

### Exemple 3

| | |
|---|---|
| Extrait de myrtille sauvage* | 1% |
| DURO-TAK 87-2979** | 85% |
| Glycerine | 5% |
| Diméthicone | 9% |

| | |
|---|---|
| * Extrait de Myrtille Herbasol SA Cosmetochem International Ltd. Sennweidstrasse 44/46 CH-6312 Steinhausen /ZG. | |
| **adhésif sensible à la pression | |

### Exemple 4

| | |
|---|---|
| Extrait de vin rouge (titré à 20% OPC) | 1 % |
| Sodium polyacrylate | 5% |
| Polyacrylic acid | 5% |
| Tartaric acid | 2% |
| Tween 80 | 3% |
| Carboxymethylcellulose | 10% |
| Glycerine | 30% |
| Eau qsp | 100% |

Le gel est obtenu par mélange des constituants puis il est enduit sous une épaisseur constante sur un support en non-tissé. Après réticulation du gel pendant 24 h, les patchs sont découpés. Un tel patch peut être appliqué 6 à 8 hrs sur la cuisse ou le bras.

### Exemple 5

La peau est préalablement nettoyée et dégraissée avec un savon et rincée avec une eau bien chaude. De suite est appliquée un patch : 1 mL d'une solution aqueuse (tampon citrate phosphate sans calcium PBS-) d'extrait de thé vert * à saturation (au moins 5 %), pH 5.5 est imbibée sur le non-tissé recouvrant la cathode du système iontophorétique IONTOPATCH® ( Travanti Pharma, St-Paul, MN, USA). Le non-tissé recouvrant l'anode est imbibé de solution saline isotonique. Le courant nominal délivré est inférieur à 100 µA. Le patch est alors appliqué sur l'avant-bras ou la cuisse pendant 2 hrs.
Dans le cas d'expositions solaires répétées on appliquera le patch dans les heures précédant l'exposition.
*: Extrait de thé vert Givaudan SA Givaudan Arome SAS BP50250 95956 Roissy Charles De Gaulle cedex

### Exemple 6

1 mL d'une solution aqueuse d'extrait de myrtille à 2% est appliquée sur une zone d'environ 5 cm2 sur la face extérieure de la cuisse. Immédiatement après on applique sur cette même zone sous une légère pression pendant 1 minute un patch de 4 cm2 constitué d'un réseau de micro-aiguilles en silicium de 100 µ de longueur (Nanopass, Israël). Les micro-trous ainsi créés permettent une pénétration optimale de l'extrait de myrtille. On utilise le patch en cures de quelques semaines particuliérement dans les périodes d'inactivité physique ou de repas copieux.

## Revendications

1. Procédé de traitement cosmétique pour prévenir et/ou retarder et/ou limiter les signes du vieillissement de la peau, des muqueuses ou des phanères, **caractérisé en ce qu'**on applique sur une zone de la peau, des muqueuses ou du cuir chevelu un système de délivrance transdermique, la composition dudit système transdermique contenant au moins un micronutriment non indispensable.

2. Procédé de traitement cosmétique selon la revendication 1, **caractérisé en ce que** le système de délivrance transdermique est appliqué dans une zone au moins partiellement distante de celle dans laquelle les signes de vieillissement sont traités.

3. Procédé de traitement cosmétique selon l'une des revendications 1 ou 2, **caractérisé en ce que** la masse moléculaire du micronutriment est inférieure ou égale à 800.

4. Procédé selon au moins l'une des revendications 1 à 3, **caractérisé en ce que** le coefficient de partage P du micronutriment entre l'octanol et l'eau est tel que log P est supérieur ou égal à -2.

5. Procédé selon au moins l'une des revendications 1 à 4, **caractérisé en ce que** le coefficient de partage P du micronutriment entre l'octanol et l'eau est tel que log P est inférieur ou égal à 5.

6. Procédé de traitement cosmétique selon au moins l'une des revendications 1 à 5, **caractérisé en ce qu'**au moins un micronutriment non indispensable est choisi dans le groupe comprenant les flavonols, les flavanols, les proanthocyanydines, les flavanones, les isoflavones, les flavonoïdes, les isoflavonoïdes, les stilbènes, leurs dérivés et analogues.

7. Procédé de traitement cosmétique selon au moins l'une des revendications 1 à 6, **caractérisé en ce qu'**au moins un micronutriment est choisi parmi, les catéchines, la théaflavine, la nobilétine, le resvératrol, les dérivés glycosylés du resvératrol, le β carotène, le lycopéne, la lutéine, la zéaxanthine, l'apigénine .

8. Procédé de traitement cosmétique selon au moins l'une des revendications 1 à 7, **caractérisé en ce que** le micronutriment est présent dans la composition sous forme d'un extrait végétal le contenant.

9. Procédé de traitement cosmétique selon au moins l'une des revendications 1 à 8, **caractérisé en ce qu'**il s'agit d'un procédé pour prévenir et/ou retarder les signes du vieillissement chronobiologique et/ou induit.

10. Procédé de traitement cosmétique selon l'une au moins des revendications 1 à 9, **caractérisé en ce que** le dispositif de délivrance transdermique est sous forme de patch, de feuilles, de film composite ou de film solide.

11. Procédé de traitement cosmétique selon l'une au moins des revendications 1 à 10, **caractérisé en ce que** le dispositif de délivrance transdermique contient en outre au moins un composé accélérateur de pénétration.

12. Procédé de traitement cosmétique selon au moins l'une des revendications 1 à 11, **caractérisé en ce que** le composé accélérateur de pénétration est choisi dans le groupe comprenant les mono- et di-alcools non cycliques, l'acétate d'éthyle, l'acétate de butyle, le myristate d'isopropyle, les acides gras, les phospholipides, les terpènes, l'azone et dérivés, le propylène glycol et dérivés glycoliques, les cyclodextrines, l'octylsalicylate, le cyclopentadecanolide, les polysorbates, la polyvinylpyrrolidone et dérivés.

13. Procédé de traitement cosmétique selon au moins l'une des revendications 1 à 12, **caractérisé en ce que** le dispositif transdermique contient en outre au moins un composé choisi parmi les gélifiants, les polymères adhésifs, les stabilisants, les colorants, les pigments, les charges, les solvants, les ions...

14. Utilisation d'au moins un micronutriment tel que défini dans au moins l'une des revendications 1 à 8, pour la préparation d'un dispositif de délivrance transdermique destiné à prévenir et/ou retarder les altérations dues au vieillissement de la peau, du cuir chevelu et/ou des phanères.

15. Dispositif de délivrance transdermique susceptible d'être utilisé dans le procédé selon l'une des revendications 1 à 13, **caractérisé en ce qu'**il s'agit d'un patch choisi parmi les patchs à libération contrôlée, les patchs réservoirs, les patchs à effet de champ magnétique, les gels patchs à base d'hydrocolldide, les gels patchs huileux et les films composites de type réservoir, et **en ce qu'**il contient de la théaflavine à une concentration comprise entre 0,001 et 10 g/cm³ de composition.
